# EUROPEAN PATENT APPLICATION

(11) **EP 4 606 374 A1**
(43) Date of publication of application: **27.08.2025**
(21) Application number: 23894016.7
(22) Date of filing: 24.11.2023
(51) Int. Cl.: A61K 31/4245, A61K 31/4422, A61K 9/20, A61K 9/16, A61K 9/48, A61K 47/38, A61P 9/00, A61P 9/12

(54) **PHARMACEUTICAL COMPOSITION COMPRISING AZILSARTAN MEDOXOMIL POTASSIUM AND CALCIUM CHANNEL BLOCKER, METHOD FOR PREPARING SAME, AND USE THEREOF**

(30) Priority: 24.11.2022 CN 202211484130
(71) Applicant: Shanghai Aurora Biotechnology Co., Ltd., Shanghai 201210 (CN)
(72) Inventor: YING, Shuhuan, Shanghai 201210 (CN); GUO, Zhen, Shanghai 201210 (CN); XIE, Wenfeng, Shanghai 201210 (CN); ZHU, Senfa, Shanghai 201210 (CN); WANG, Tingting, Shanghai 201210 (CN)
(74) Representative: Gille Hrabal Partnerschaftsgesellschaft mbB Patentanwälte
(86) International application number: PCT/CN2023/133949
(87) International publication number: WO 2024/109927

(57) **Abstract**

The present invention provides a pharmaceutical composition comprising azilsartan medoxomil potassium and a calcium channel blocker, a method for preparing same, and use thereof. Azilsartan medoxomil potassium and the calcium channel blocker are formulated into a compound formulation, thus improving the blood pressure lowering efficacy, improving safety and tolerability, and reducing adverse effects. Azilsartan medoxomil potassium can ameliorate peripheral edema caused by amlodipine by means of expanding peripheral venous vessels, while amlodipine can ameliorate adverse effects such as vasogenic edema, dry cough, and the like caused by azilsartan medoxomil potassium. Compared with the monotherapies, the combination has the advantages of (1) reducing the number of doses and thus improving patient compliance; (2) improving the compliance in seniors or those with dysphagia; (3) preventing frequent fluctuation in plasma drug concentration, maintaining a stable plasma concentration, maintaining the long-term stability of blood pressure in patients with hypertension, and reducing the adverse effects of the drugs; and (4) preventing arbitrary treatment interruption, and thus preventing disease recurrence and the development of malignant complications.

## Description

The present application claims priority to the prior Chinese Patent Application for Invention with the application No. 202211484130.8 and entitled "PHARMACEUTICAL COMPOSITION OF AZILSARTAN MEDOXOMIL POTASSIUM AND CALCIUM CHANNEL BLOCKER, PREPARATION METHOD THEREFOR, AND USE THEREOF" filed with the China National Intellectual Property Administration by the applicant on November 24, 2022, which is incorporated herein by reference in its entirety.

### TECHNICAL FIELD

The present disclosure relates to a pharmaceutical composition comprising azilsartan medoxomil potassium and a calcium channel blocker, a preparation method therefor, and use thereof.

### BACKGROUND

Hypertension is one of the most common cardiovascular diseases, affecting 25%-30% of the adult population. If left uncontrolled over the long run, hypertension can increase the risk of developing coronary atherosclerosis, left ventricular hypertrophy, carotid atherosclerosis, kidney disease, and the like. The survey results from the Report on Cardiovascular Health and Diseases in China (2019) revealed that the number of hypertension patients in China has reached 245 million, with an adult prevalence rate of about 27.9%, showing a persistent upward trend year by year. However, the hypertension control rate in China remains at only 16.8%, and a significant proportion of the hypertensive population has not achieved effective blood pressure control. Therefore, the development of pharmaceutical formulations for the treatment of hypertension and cardiovascular diseases with reduced toxic and side effects is of positive significance for clinical medication.

Azilsartan medoxomil potassium is an angiotensin II receptor antagonist (ARB) drug developed by Takeda Pharmaceuticals. As a prodrug, this drug is rapidly converted to the active ingredient azilsartan following oral absorption. Azilsartan selectively blocks the binding of angiotensin II to the AT1 receptor in various tissues, thereby blocking the effects of angiotensin II. Angiotensin II is the main pressor substance of RAAS, and has effects including vasoconstriction, stimulation of aldosterone synthesis and release, cardiac excitation, renal sodium reabsorption, and the like. Azilsartan medoxomil potassium selectively blocks the binding of angiotensin II to the AT1 receptor in various tissues (e.g., vascular smooth muscle and adrenal glands), thereby blocking the vasoconstrictive effect and aldosterone secretion effect of angiotensin II.

The chemical name of azilsartan medoxomil potassium is (5-methyl-2-oxo-1,3-dioxol-4-yl)methyl 2-ethoxy-1-{[2'-(5-oxo-4,5-dihydro-1,2,4-oxadiazol-3-yl)biphenyl-4-yl]methyl}-1H-benzimidazole-7-carboxylate monopotassium salt, its molecular weight is 606.62, and its structural formula is as follows: 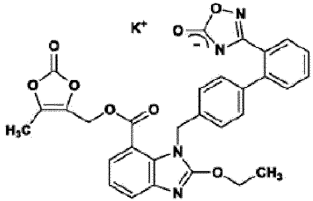 (azilsartan medoxomil potassium)

Calcium channel blockers, also known as calcium antagonists, are a class of drugs that selectively act on calcium channels to block the influx of extracellular calcium ions into cells, thereby affecting cellular functions. Intracellular calcium ions play a critical role in cellular functions: as an important intracellular second messenger, they regulate various cellular responses and activities, including neurotransmitter release, muscle contraction, glandular secretion, platelet activation, and the like, and particularly exert a significant effect on the function of the cardiovascular system.

Amlodipine is one type of calcium ion channel blocker. Since the contraction of myocardial and smooth muscle depends on the influx of extracellular calcium ions into cells through specific ion channels, amlodipine selectively inhibits the transmembrane influx of calcium ions into smooth muscle cells and myocardial cells, with a greater effect on smooth muscle than on myocardial cells. The interaction between amlodipine and calcium channels is determined by the gradual rates of its binding to and dissociation from receptor sites, and therefore, its pharmacological effects develop gradually. Amlodipine is a peripheral arterial vasodilator that directly acts on vascular smooth muscle, reduces peripheral vascular resistance, and thereby lowers blood pressure. The chemical name of amlodipine is ethyl methyl 6-methyl-2-(2-aminoethoxy)methyl-4-(2-chlorophenyl)-1,4-dihydro-3,5-pyridinedicarboxylate, its molecular weight is 408.88, and its structural formula is as follows:

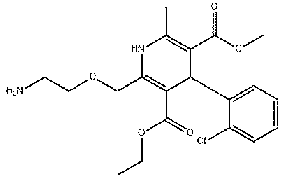

Levamlodipine is a dihydropyridine calcium channel blocker that directly acts on peripheral arterial vascular smooth muscle, reduces peripheral vascular resistance, and thereby lowers blood pressure. Levamlodipine blocks the influx of extracellular calcium ions into myocardial and vascular smooth muscle cells through calcium ion channels (slow channels) in the cell membrane and directly relaxes vascular smooth muscle to reduce blood pressure. Simultaneously, levamlodipine reduces the vasoconstrictive effect of angiotensin II and adrenergic receptors and decreases renal tubular reabsorption of sodium.

Currently known commonly reported adverse effects of azilsartan medoxomil potassium include dizziness, hypotension, diarrhea, angioedema, and the like; commonly reported adverse effects of amlodipine include arrhythmia, bradycardia, chest pain, hypotension, peripheral ischemia, syncope, and the like. Therefore, there is a need to develop a pharmaceutical composition comprising azilsartan medoxomil potassium and a calcium channel blocker, which exhibits reduced side effects and improved compliance, a preparation method, and use.

### SUMMARY

The present disclosure aims to provide a pharmaceutical composition comprising azilsartan medoxomil potassium and a calcium channel blocker, which exhibits reduced side effects and improved compliance and is distinct from the prior art, a preparation method therefor, and use thereof.

The present disclosure provides a pharmaceutical composition comprising azilsartan medoxomil potassium and a calcium channel blocker, wherein:
the azilsartan medoxomil potassium comprises one or more of azilsartan medoxomil potassium, a pharmaceutically acceptable salt thereof, and a solvate thereof; and
the calcium channel blocker comprises one or more of a calcium channel blocker, a pharmaceutically acceptable salt thereof, and a solvate thereof.

According to an embodiment of the present disclosure, the calcium channel blocker may be one or more of amlodipine, levamlodipine, and a pharmaceutically acceptable salt thereof (e.g., amlodipine besylate or levamlodipine besylate).

According to an embodiment of the present disclosure, a content of azilsartan medoxomil potassium, the pharmaceutically acceptable salt thereof, and/or the solvate thereof comprised in the pharmaceutical composition may be 20 mg-80 mg, such as 20 mg, 40 mg, or 80 mg. Preferably, the content of azilsartan medoxomil potassium, the pharmaceutically acceptable salt thereof, and/or the solvate thereof in a unit dose form of the pharmaceutical composition may be 20 mg-80 mg, such as 20 mg, 40 mg, or 80 mg.

Alternatively, based on a total weight of the pharmaceutical composition, a mass percentage content of azilsartan medoxomil potassium, the pharmaceutically acceptable salt thereof, and/or the solvate thereof comprised in the pharmaceutical composition may be 1%-50%, such as 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, or 50%, such as 14.23%, 21.33%, or 21.34%.

According to an embodiment of the present disclosure, a content of the calcium channel blocker, the pharmaceutically acceptable salt thereof, and/or the solvate thereof comprised in the pharmaceutical composition may be 2.5 mg-20 mg, such as 2.5 mg-20 mg, preferably 2.5 mg, 5 mg, 7.5 mg, 10 mg, or 20 mg. Alternatively, a content of levamlodipine comprised in the pharmaceutical composition is 2.5 mg-10 mg, such as 2.5 mg, 5 mg, 7.5 mg, 10 mg, or 20 mg, preferably 2.5 mg, 5 mg, or 10 mg. Preferably, the content of the calcium channel blocker, the pharmaceutically acceptable salt thereof, and/or the solvate thereof in a unit dose form of the pharmaceutical composition may be 2.5 mg-20 mg, such as 2.5 mg-20 mg, preferably 2.5 mg, 5 mg, 7.5 mg, 10 mg, or 20 mg. Alternatively, the content of levamlodipine in a unit dose form of the pharmaceutical composition is 2.5 mg-10 mg, such as 2.5 mg, 5 mg, 7.5 mg, 10 mg, or 20 mg, preferably 2.5 mg, 5 mg, or 10 mg.

Alternatively, based on the total weight of the pharmaceutical composition, a mass percentage content of the calcium channel blocker, the pharmaceutically acceptable salt thereof, and/or the solvate thereof comprised in the pharmaceutical composition may be 0.5%-10%, such as 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.5%, 3%, 3.5%, 4%, 4.5%, 5%, 5.5%, 6%, 6.5%, 7%, 7.5%, 8%, 8.5%, 9%, 9.5%, or 10%, such as 1.74%, 2.31%, or 3.47%.

According to an embodiment of the present disclosure, in the pharmaceutical composition, a ratio of the total weight of azilsartan medoxomil potassium, the pharmaceutically acceptable salt thereof, and/or the solvate thereof to the total weight of the calcium channel blocker, the pharmaceutically acceptable salt thereof, and/or the solvate thereof may be 1-32: 1, and may be 1:1, 2:1, 2.7:1, 4:1, 5.3:1, 8:1, 16:1, or 32:1.

According to an embodiment of the present disclosure, the pharmaceutical composition may further comprise one or more of a pH regulator, a diluent, an adhesive, a disintegrant, a lubricant, a glidant, a tableting aid, a colorant, and a coating premix.

According to an embodiment of the present disclosure, the pH regulator refers to a substance for regulating the pH of the composition and is selected from one or more of fumaric acid, sodium fumarate, citric acid, sodium citrate, tartaric acid, sodium hydroxide, potassium dihydrogen phosphate, sodium dihydrogen phosphate, dicalcium phosphate, and sodium carbonate. A mass percentage content of the pH regulator is preferably 0-10%, such as 0.1%, 0.2%, 0.3%, 0.4%, 0.5%, 0.6%, 0.7%, 0.8%, 0.9%, 1%, 1.1%, 1.2%, 1.3%, 1.4%, 1.5%, 1.6%, 1.7%, 1.8%, 1.9%, 2%, 2.5%, 3%, 3.5%, 4%, 4.5%, 5%, 5.5%, 6%, 6.5%, 7%, 7.5%, 8%, 8.5%, 9%, 9.5%, or 10%, wherein the mass percentage content refers to the percentage of the mass of the pH regulator in the total mass of the composition of azilsartan medoxomil potassium and the calcium channel blocker.

According to an embodiment of the present disclosure, the diluent refers to a solid substance capable of improving the performance of a material, increasing the volume and weight, and reducing the cost of the material when added to the material and is selected from one or more of mannitol, anhydrous dicalcium phosphate, sucrose, glucose, maltose, lactose (e.g., lactose monohydrate), microcrystalline cellulose, and sorbitol. A mass percentage content of the diluent is preferably 0-80%, such as 5%, 10%, 15%, 20%, 25%, 30%, 35%, 40%, 45%, 50%, 54%, 54.63%, 55%, 60%, 64%, 64.63%, 65%, 70%, 75%, or 80%, wherein the mass percentage content refers to the percentage of the mass of the diluent in the total mass of the composition of azilsartan medoxomil potassium and the calcium channel blocker.

According to an embodiment of the present disclosure, the adhesive (also referred to as a thickener) may be an additive known in the art that is capable of increasing the viscosity of a dispersion medium to reduce the settling rate of microparticles or increase the hydrophilicity of microparticles and is selected from one or more of hydroxypropylcellulose, hydroxypropyl methylcellulose, hydroxyethylcellulose, copovidone, povidone, and pregelatinized starch. A mass percentage content of the adhesive is preferably 0-20%, such as 0.5%, 1%, 2%, 3%, 4%, 5%, 6%, 7%, 8%, 9%, 10%, 11%, 12%, 13%, 14%, 15%, 16%, 17%, 18%, 19%, or 20%, wherein the mass percentage content refers to the percentage of the mass of the adhesive in the total mass of the composition of azilsartan medoxomil potassium and the calcium channel blocker.

According to an embodiment of the present disclosure, the disintegrant refers to a substance that is capable of disintegrating a tablet or granule in dissolution and is selected from one or more of sodium carboxymethyl starch, croscarmellose sodium, low-substituted hydroxypropylcellulose, crospovidone, and corn starch. A mass percentage content of the disintegrant is preferably 0-20%, such as 0.5%, 1%, 2%, 3%, 4%, 5%, 6%, 7%, 8%, 9%, 10%, 11%, 12%, 13%, 14%, 15%, 16%, 17%, 18%, 19%, or 20%, wherein the mass percentage content refers to the percentage of the mass of the disintegrant in the total mass of the composition of azilsartan medoxomil potassium and the calcium channel blocker.

According to an embodiment of the present disclosure, the lubricant may be a substance known in the art that has a lubricative effect and is selected from one or more of a metal stearate (e.g., magnesium stearate), stearic acid, talc, a stearic acid ester, stearyl fumarate, and micronized silica gel. A mass percentage content of the lubricant is preferably 0-10%, such as 0.5%, 1%, 2%, 3%, 4%, 5%, 6%, 7%, 8%, 9%, or 10%, wherein the mass percentage content refers to the percentage of the mass of the lubricant in the total mass of the composition of azilsartan medoxomil potassium and the calcium channel blocker.

According to an embodiment of the present disclosure, the glidant may be an auxiliary material known in the art that is capable of reducing the friction between particles to improve the flowability of powders or granules and is selected from one or more of talc, micronized silica gel, colloidal silicon dioxide, and pregelatinized starch. A mass percentage content of the glidant is preferably 0-10%, wherein the mass percentage content refers to the percentage of the mass of the glidant in the total mass of the composition of azilsartan medoxomil potassium and the calcium channel blocker.

According to an embodiment of the present disclosure, the tableting aid is an auxiliary material that is capable of improving flowability and/or compressibility and increasing tablet hardness and wear resistance and is selected from microcrystalline cellulose and/or lactose monohydrate. According to an embodiment of the present disclosure, the colorant may be a substance known in the art that is capable of achieving coloring purposes and is selected from iron oxide red and/or iron oxide yellow. A mass percentage content of the colorant is preferably 0-5%, wherein the mass percentage content refers to the percentage of the mass of the colorant in the total mass of the composition of azilsartan medoxomil potassium and the calcium channel blocker.

According to an embodiment of the present disclosure, the coating premix is known in the prior art. For example, the coating premix is a film coating premix, which comprises one or more of hydroxypropyl methylcellulose, polyethylene glycol, polyvinyl alcohol, lactose, titanium dioxide, red iron oxide, yellow iron oxide, and black iron oxide. As an example, the coating premix may be selected from a film coating premix provided by Colorcon.

The pharmaceutical composition of the present disclosure may be a composition comprising azilsartan medoxomil potassium and amlodipine (e.g. an azilsartan medoxomil potassium-amlodipine tablet) or a composition comprising azilsartan medoxomil potassium and levamlodipine (e.g. an azilsartan medoxomil potassium-levamlodipine tablet).

According to an embodiment of the present disclosure, azilsartan medoxomil potassium and the calcium channel blocker in the pharmaceutical composition may be distributed in a single-layer drug core structure or a double-layer drug core structure. When the pharmaceutical composition is a single-layer drug core structure, the single-layer drug core comprises azilsartan medoxomil potassium and the calcium channel blocker; or, when the pharmaceutical composition is a double-layer drug core structure, one layer of the drug core comprises azilsartan medoxomil potassium, and the other layer of the drug core comprises the calcium channel blocker.

When the pharmaceutical composition is a double-layer drug core structure, the drug core layer comprising azilsartan medoxomil potassium and the drug core layer comprising the calcium channel blocker may independently comprise one or more of the pH regulator, the diluent, the adhesive, the disintegrant, the lubricant, the glidant, the tableting aid, the colorant, and the coating premix described above.

For example, the drug core layer comprising azilsartan medoxomil potassium may independently comprise one or more of the pH regulator, the diluent, the adhesive, the disintegrant, the lubricant, and the colorant described above; the drug core layer comprising the calcium channel blocker may independently comprise one or more of the diluent, the disintegrant, the lubricant, the glidant, and the colorant described above.

According to an embodiment of the present disclosure, the pharmaceutical composition may be any one of the following formulas:
formula one: 21.33% azilsartan medoxomil potassium, 3.47% amlodipine besylate, 54.00% mannitol, 0.80% fumaric acid, 0.40% sodium hydroxide, 3.00% hydroxypropylcellulose, 6.00% sodium carboxymethyl starch, 10.00% microcrystalline cellulose, and 1.00% magnesium stearate, wherein each of the percentages is the percentage of the weight of the corresponding component in the total mass of the composition of azilsartan medoxomil potassium and the calcium channel blocker;
formula two: 21.33% azilsartan medoxomil potassium, 1.74% levamlodipine besylate, 54.63% mannitol, 0.80% fumaric acid, 0.50% sodium hydroxide, 3.00% hydroxypropylcellulose, 7.00% sodium carboxymethyl starch, 10.00% microcrystalline cellulose, and 1.00% magnesium stearate, wherein each of the percentages is the percentage of the weight of the corresponding component in the total mass of the composition of azilsartan medoxomil potassium and the calcium channel blocker;
formula three:
   an amlodipine layer: 6.93% amlodipine besylate, 58.07% microcrystalline cellulose, 30.00% lactose monohydrate, 4.00% sodium carboxymethyl starch, and 1.00% magnesium stearate, wherein each of the percentages is the percentage of the weight of the corresponding component in the total mass of the amlodipine layer; and
   an azilsartan medoxomil potassium layer: 21.34% azilsartan medoxomil potassium, 52.31% mannitol, 6.00% croscarmellose sodium, 3.00% hydroxypropylcellulose, 1.00% fumaric acid, 0.35% sodium hydroxide, 15.00% microcrystalline cellulose, and 1.00% magnesium stearate, wherein each of the percentages is the percentage of the weight of the corresponding component in the total mass of the azilsartan medoxomil potassium layer;
formula four:
   an amlodipine layer: 6.93% amlodipine besylate, 66.07% microcrystalline cellulose, 20.00% anhydrous dicalcium phosphate, 6.00% sodium carboxymethyl starch, and 1.00% magnesium stearate, wherein each of the percentages is the percentage of the weight of the corresponding component in the total mass of the amlodipine layer; and
   an azilsartan medoxomil potassium layer: 21.34% azilsartan medoxomil potassium, 25.00% lactose monohydrate, 38.31% mannitol, 7.00% hydroxypropylcellulose, 1.00% fumaric acid, 0.35% sodium hydroxide, 6.00% sodium carboxymethyl starch, and 1.00% magnesium stearate, wherein each of the percentages is the percentage of the weight of the corresponding component in the total mass of the azilsartan medoxomil potassium layer;
formula five:
   21.34% azilsartan medoxomil potassium, 50.05% mannitol, 1.00% fumaric acid, 0.35% sodium hydroxide, 2.70% hydroxypropylcellulose, 3.47% amlodipine besylate, 12.50% microcrystalline cellulose, 7.50% croscarmellose sodium, and 1.10% magnesium stearate, wherein each of the percentages is the percentage of the weight of the corresponding component in the total mass of the composition of azilsartan medoxomil potassium and the calcium channel blocker;
formula six:
   21.34% azilsartan medoxomil potassium, 50.19% mannitol, 0.87% fumaric acid, 0.35% sodium hydroxide, 2.70% hydroxypropylcellulose, 3.47% amlodipine besylate, 12.50% microcrystalline cellulose, 7.50% croscarmellose sodium, and 1.10% magnesium stearate, wherein each of the percentages is the percentage of the weight of the corresponding component in the total mass of the composition of azilsartan medoxomil potassium and the calcium channel blocker;
formula seven or eight:
   21.34% azilsartan medoxomil potassium, 51.00% mannitol, 0.88% fumaric acid, 0.33% sodium hydroxide, 2.00% hydroxypropylcellulose, 3.47% amlodipine besylate, 12.50% microcrystalline cellulose, 7.50% croscarmellose sodium, and 1.10% magnesium stearate, wherein each of the percentages is the percentage of the weight of the corresponding component in the total mass of the composition of azilsartan medoxomil potassium and the calcium channel blocker;
formula nine:
   an amlodipine layer: 2.31% amlodipine besylate, 33.33% microcrystalline cellulose, 3.69% croscarmellose sodium, and 0.67% magnesium stearate, wherein each of the percentages is the percentage of the weight of the corresponding component in the total mass of the amlodipine layer; and
   an azilsartan medoxomil potassium layer: 14.23% azilsartan medoxomil potassium, 33.97% mannitol, 1.33% hydroxypropylcellulose, 0.58% fumaric acid, 0.22% sodium hydroxide, 5.00% microcrystalline cellulose, 4.00% croscarmellose sodium, and 0.67% magnesium stearate, wherein each of the percentages is the percentage of the weight of the corresponding component in the total mass of the azilsartan medoxomil potassium layer.

The present disclosure further provides a preparation method for the pharmaceutical composition, which includes, but is not limited to, subjecting the components described above to a method selected from a direct powder compression method, a wet granulation method, a fluidized granulation method, a dry granulation method, an extrusion-spheronization method, and a pellet coating method to prepare the pharmaceutical composition.

The present disclosure further provides use of the pharmaceutical composition for treating and/or preventing a cardiovascular disease. The present disclosure further provides a method of treatment and/or prevention of a cardiovascular disease, which comprises administering to a mammal (e.g., a human) in need thereof a therapeutically effective amount of the pharmaceutical composition.

The present disclosure further provides use of the pharmaceutical composition for manufacturing a medicament or a pharmaceutical formulation.

According to an embodiment of the present disclosure, the medicament or pharmaceutical formulation is for use in the treatment and/or prevention of a cardiovascular disease.

According to an embodiment of the present disclosure, the pharmaceutical formulation may be an oral pharmaceutical formulation. For example, a dosage form of the pharmaceutical formulation includes, but is not limited to, a compound tablet, a double-layer tablet, a capsule, a pellet, a micro-tablet, and the like.

According to an embodiment of the present disclosure, the cardiovascular disease is selected from, for example, at least one of hypertension, heart failure, coronary heart disease, angina, arrhythmia, myocardial infarction, congenital heart disease, heart valve disease, and the like.

The reagents and starting materials used in the present disclosure are commercially available.

### Beneficial Effects

In the present disclosure, azilsartan medoxomil potassium and the calcium channel blocker are in combined use and formulated into a pharmaceutical composition or a compound formulation, which is conducive to enhancing antihypertensive effects, improving safety and tolerability, and reducing side effects. In the pharmaceutical composition or the compound formulation, azilsartan medoxomil potassium can alleviate peripheral edema caused by amlodipine through peripheral venous vasodilation, while amlodipine can alleviate adverse effects such as angioedema, dry cough, and the like caused by azilsartan medoxomil potassium. Compared with monotherapy with individual active ingredients, the advantages of the pharmaceutical composition of the present disclosure include at least: (1) reducing the number of pills that need to be taken and thus improving patient compliance; (2) helping improve adherence for elderly people or people with swallowing difficulties; (3) preventing frequent fluctuations in plasma concentrations, thereby maintaining stable plasma concentrations *in vivo,* maintaining stable blood pressures in patients with hypertension over the long run, and reducing the side effects of each of the drugs; and (4) preventing patients from interrupting medication at will and preventing disease recurrence and progression of severe complications.

### BRIEF DESCRIPTION OF THE DRAWINGS

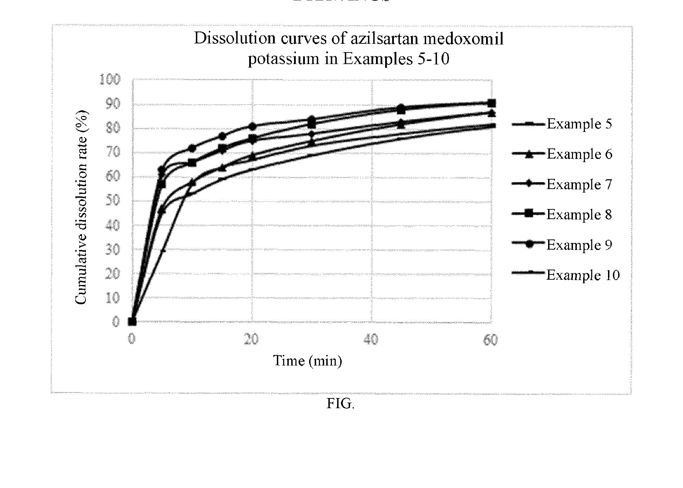

FIG. 1 shows the dissolution curves of azilsartan medoxomil potassium in the formulas in Examples 5-10 in a phosphate buffer medium at pH 7.2.

### DETAILED DESCRIPTION

The present disclosure is further illustrated by the following examples; however, these examples should not be construed as limiting the present disclosure. Experimental procedures without specified conditions in the following examples were conducted in accordance with conventional procedures and conditions, or in accordance with product instructions.

Unless otherwise specified, the film coating premix mentioned hereinafter is a film coating premix provided by Colorcon.

### Example 1

### 1) Formula composition

| Name | Feeding amount per tablet (mg) | Percentage (%) |
|---|---|---|
| Azilsartan medoxomil potassium | 42.68 | 21.33 |
| Amlodipine besylate | 6.93 | 3.47 |
| Mannitol | 107.99 | 54.00 |
| Fumaric acid | 1.6 | 0.80 |
| Sodium hydroxide | 0.8 | 0.40 |
| Hydroxypropylcellulose | 6 | 3.00 |
| Sodium carboxymethyl starch | 12 | 6.00 |
| Microcrystalline cellulose | 20 | 10.00 |
| Magnesium stearate | 2 | 1.00 |
| Film coating premix | 8 | / |

### 2) Preparation method

Adhesive preparation: A solution with a solid content of 5% (the percentage refers to the percentage of the weight of hydroxypropylcellulose, fumaric acid, and sodium hydroxide in the total weight of the solution) was prepared from hydroxypropylcellulose, fumaric acid, sodium hydroxide, and purified water. The mixture was stirred homogeneously and set aside for later use.

Azilsartan medoxomil potassium, amlodipine besylate, and mannitol were placed in a hopper mixer and mixed for 10 min. The mixture was then placed into a fluidized bed, and parameters including the inlet airflow rate, the inlet air temperature, the atomization temperature, the peristaltic pump speed, and the like were set. The aqueous adhesive solution was added using a peristaltic pump, and fluidized granulation was performed with simultaneous drying. The granulated material was passed through a 1.5-mm sieve. The dry granules were added to a mixing hopper, and sodium carboxymethyl starch and microcrystalline cellulose were added. The materials were mixed for 10 min. Magnesium stearate was then added, and the materials were mixed for 5 min to give azilsartan medoxomil potassium-amlodipine final mixed granules.

The azilsartan medoxomil potassium-amlodipine final mixed granules were placed into a rotary tablet press and tableted using round shallow-arc punches. The resulting azilsartan medoxomil potassium-amlodipine plain tablets were coated with an aqueous solution of a film coating premix (solid content: 13%) (the percentage refers to the percentage of the weight of the film coating premix in the total mass of the aqueous solution of the film coating premix) to give azilsartan medoxomil potassium-amlodipine tablets.

### Example 2

| Name | Feeding amount per tablet (mg) | Percentage (%) |
|---|---|---|
| Azilsartan medoxomil potassium | 42.68 | 21.33 |
| Levamlodipine besylate | 3.47 | 1.74 |
| Mannitol | 98.86 | 54.63 |
| Microcrystalline cellulose | 20 | 10.00 |
| Fumaric acid | 1.6 | 0.80 |
| Sodium hydroxide | 1.0 | 0.50 |
| Hydroxypropylcellulose | 6 | 3.00 |
| Croscarmellose sodium | 14 | 7.00 |
| Magnesium stearate | 2 | 1.00 |
| Film coating premix | 8 | / |

### 2) Preparation method

Adhesive preparation: A solution with a solid content of 5% (the percentage refers to the percentage of the weight of hydroxypropylcellulose, fumaric acid, and sodium hydroxide in the total weight of the solution) was prepared from hydroxypropylcellulose, fumaric acid, sodium hydroxide, and purified water. The mixture was stirred homogeneously and set aside for later use.

Azilsartan medoxomil potassium, levamlodipine besylate, mannitol, microcrystalline cellulose, and croscarmellose sodium were added to a wet granulation mixing vessel. The stirring speed and the cutting speed were activated, and the materials were mixed for 10 min. An aqueous solution of hydroxypropylcellulose, fumaric acid, and sodium hydroxide was then added, and wet granulation was performed. The granulated material was wet-sized using a 1.5-mm sieve. The sized material was placed into an oven and dried until the water content was less than 3.0%.

The dried granules were passed through a 1.2-mm sieve for dry sizing to give azilsartan medoxomil potassium-levamlodipine dry granules.

The azilsartan medoxomil potassium-levamlodipine dry granules were added to a mixing vessel. Magnesium stearate was added, and the materials were mixed for 5 min. The mixture was then tableted using a rotary tablet press. The resulting azilsartan medoxomil potassium-levamlodipine plain tablets were coated with an aqueous solution of a film coating premix (solid content: 13%) (the percentage refers to the percentage of the weight of the film coating premix in the total mass of the aqueous solution of the film coating premix) to give azilsartan medoxomil potassium-levamlodipine tablets.

### Example 3

### 1) Formula composition

| Name | Feeding amount per tablet (mg) | Percentage (%) |
|---|---|---|
| **Amlodipine layer** | | |
| Amlodipine besylate | 6.93 | 6.93 |
| Microcrystalline cellulose | 58.07 | 58.07 |
| Lactose monohydrate | 30 | 30 |
| Sodium carboxymethyl starch | 4 | 4 |
| Magnesium stearate | 1 | 1 |

| **Azilsartan medoxomil potassium layer** | | |
|---|---|---|
| Azilsartan medoxomil potassium | 42.68 | 21.34 |
| Mannitol | 104.62 | 52.31 |
| Croscarmellose sodium | 12 | 6.00 |
| Hydroxypropylcellulose | 6 | 3.00 |
| Fumaric acid | 2 | 1.00 |
| Sodium hydroxide | 0.7 | 0.35 |
| Microcrystalline cellulose | 30 | 15.00 |
| Magnesium stearate | 2 | 1.00 |

| **Coating** | | |
|---|---|---|
| Film coating premix | 12 | / |

### 2) Preparation method

Amlodipine layer final mixed material preparation:
Amlodipine besylate, microcrystalline cellulose, lactose monohydrate, and sodium carboxymethyl starch were placed into a three-dimensional mixer and mixed for 20 min. A dry granulator was used; the rolling pressure, the rolling speed, and the feeding rate were adjusted for dry granulation. The resulting granules were dry-sized using a 20-mesh sieve. Magnesium stearate was then added, and the materials were mixed for 5 min to give amlodipine final mixed granules.

Azilsartan medoxomil potassium layer final mixed material preparation:
Adhesive preparation: A solution with a solid content of 5% was prepared from hydroxypropylcellulose, fumaric acid, sodium hydroxide, and purified water. The mixture was stirred homogeneously and set aside for later use.

Azilsartan medoxomil potassium, mannitol, and croscarmellose sodium were added to a hopper mixer and mixed for 10 min. The mixture was then placed into a fluidized bed, and parameters including the inlet airflow rate, the inlet air temperature, the atomization temperature, the peristaltic pump speed, and the like were set. An aqueous solution of hydroxypropylcellulose, fumaric acid, and sodium hydroxide was added using a peristaltic pump, and fluidized granulation was performed with simultaneous drying. The granulated material was passed through a 1.5-mm sieve. The dry granules were added to a mixing hopper, and microcrystalline cellulose was added. The materials were mixed for 10 min. Magnesium stearate was then added, and the materials were mixed for 5 min to give azilsartan medoxomil potassium final mixed granules.

The amlodipine final mixed granules and the azilsartan medoxomil potassium final mixed granules were tableted using a double-layer tablet press to give azilsartan medoxomil potassium-amlodipine plain tablets, and film coating was then performed to give azilsartan medoxomil potassium-amlodipine tablets.

### Example 4

### 1) Formula composition

| Name | Feeding amount per tablet (mg) | Percentage (%) |
|---|---|---|
| **Amlodipine layer** | | |
| Amlodipine besylate | 6.93 | 6.93 |
| Microcrystalline cellulose | 66.07 | 66.07 |
| Anhydrous dicalcium phosphate | 20 | 20.00 |
| Sodium carboxymethyl starch | 6 | 6.00 |
| Magnesium stearate | 1 | 1.00 |

| **Azilsartan medoxomil potassium layer** | | |
|---|---|---|
| Azilsartan medoxomil potassium | 42.68 | 21.34 |
| Lactose monohydrate | 50 | 25.00 |
| Mannitol | 76.62 | 38.31 |
| Hydroxypropylcellulose | 14 | 7.00 |
| Fumaric acid | 2 | 1.00 |
| Sodium hydroxide | 0.7 | 0.35 |
| Sodium carboxymethyl starch | 12 | 6.00 |
| Magnesium stearate | 2 | 1.00 |

| **Coating** | | |
|---|---|---|
| Film coating premix | 12 | / |

### 2) Preparation method

Amlodipine layer final mixed material preparation:
Amlodipine besylate, microcrystalline cellulose, anhydrous dicalcium phosphate, and sodium carboxymethyl starch were placed into a three-dimensional mixer and mixed for 20 min. Magnesium stearate was then added, and the materials were mixed for 5 min to give amlodipine final mixed granules.

Azilsartan medoxomil potassium layer final mixed material preparation:
Adhesive preparation: A solution with a solid content of 5% was prepared from hydroxypropylcellulose, fumaric acid, sodium hydroxide, and purified water. The mixture was stirred homogeneously and set aside for later use.

Azilsartan medoxomil potassium, mannitol, and lactose monohydrate were added to a wet granulation mixing vessel. The stirring speed and the cutting speed were activated, and the materials were mixed for 10 min. An aqueous solution of hydroxypropylcellulose, fumaric acid, and sodium hydroxide was then added, and wet granulation was performed. The granulated material was wet-sized using a 1.5-mm sieve. The sized material was placed into an oven and dried until the water content was less than 3.0%. The dried granules were passed through a 1.0-mm sieve for dry sizing. The azilsartan medoxomil potassium dry granules were then added to a hopper mixer. Sodium carboxymethyl starch and magnesium stearate were added, and the materials were mixed for 5 min to give azilsartan medoxomil potassium final mixed granules.

The amlodipine final mixed material and the azilsartan medoxomil potassium final mixed granules were tableted using a double-layer tablet press to give azilsartan medoxomil potassium-amlodipine plain tablets, and film coating was then performed to give azilsartan medoxomil potassium-amlodipine tablets.

### Example 5

### 1) Formula composition

| Name | Feeding amount per tablet (mg) | Percentage (%) |
|---|---|---|
| Internal addition | | |
| Azilsartan medoxomil potassium | 42.68 | 21.34 |
| Mannitol | 100.1 | 50.05 |

| Adhesive | | |
|---|---|---|
| Fumaric acid | 2 | 1.00 |
| Sodium hydroxide | 0.69 | 0.35 |
| Hydroxypropylcellulose | 5.4 | 2.70 |

| External addition | | |
|---|---|---|
| Amlodipine besylate | 6.93 | 3.47 |
| Microcrystalline cellulose | 25 | 12.50 |
| Croscarmellose sodium | 15 | 7.50 |
| Magnesium stearate | 2.2 | 1.10 |

### 2) Preparation method

Adhesive preparation: A solution with a solid content of 5% was prepared from hydroxypropylcellulose, fumaric acid, sodium hydroxide, and purified water. The mixture was stirred homogeneously and set aside for later use. Premixing: Mannitol and azilsartan medoxomil potassium were placed in a hopper mixer and mixed for 20 min.

Granulation: The mixed mannitol and azilsartan medoxomil potassium were added to a fluidized bed. The inlet airflow rate and the inlet air temperature were set, and preheating was performed. The aqueous solution of hydroxypropylcellulose, fumaric acid, and sodium hydroxide was then added, and fluidized granulation was performed. After granulation, the material was discharged when the water content was less than 3.0%. The granulated material was sized using a 1.5-mm sieve. External final mix: The azilsartan medoxomil potassium dry granules were then added to a hopper mixer. Amlodipine besylate, microcrystalline cellulose, and croscarmellose sodium were added, and the materials were mixed for 10 min. Magnesium stearate was then added, and the materials were mixed for another 5 min to give azilsartan medoxomil potassium-amlodipine final mixed granules.

Tableting: Finally, the azilsartan medoxomil potassium-amlodipine final mixed granules were tableted using a tablet press to give azilsartan medoxomil potassium-amlodipine tablets.

### Example 6

### 1) Formula composition

| Name | Feeding amount per tablet (mg) | Percentage (%) |
|---|---|---|
| Internal addition | | |
| Azilsartan medoxomil potassium | 42.68 | 21.34 |
| Mannitol | 100.37 | 50.19 |

| Adhesive | | |
|---|---|---|
| Fumaric acid | 1.73 | 0.87 |
| Sodium hydroxide | 0.69 | 0.35 |
| Hydroxypropylcellulose | 5.4 | 2.70 |

| External addition | | |
|---|---|---|
| Amlodipine besylate | 6.93 | 3.47 |
| Microcrystalline cellulose | 25 | 12.50 |
| Croscarmellose sodium | 15 | 7.50 |
| Magnesium stearate | 2.2 | 1.10 |

### 2) Preparation method

Adhesive preparation: A solution with a solid content of 4% was prepared from hydroxypropylcellulose, fumaric acid, sodium hydroxide, and purified water. The mixture was stirred homogeneously and set aside for later use. Premixing: Mannitol and azilsartan medoxomil potassium were placed in a hopper mixer and mixed for 20 min.

Granulation: The mixed mannitol and azilsartan medoxomil potassium were added to a fluidized bed. The inlet airflow rate and the inlet air temperature were set, and preheating was performed. The aqueous solution of hydroxypropylcellulose, fumaric acid, and sodium hydroxide was then added, and fluidized granulation was performed. After granulation, the material was discharged when the water content was less than 3.0%. The granulated material was sized using a 1.5-mm sieve. External final mix: The azilsartan medoxomil potassium dry granules were then added to a hopper mixer. Amlodipine besylate, microcrystalline cellulose, and croscarmellose sodium were added, and the materials were mixed for 10 min. Magnesium stearate was then added, and the materials were mixed for another 5 min to give azilsartan medoxomil potassium-amlodipine final mixed granules.

Tableting: Finally, the azilsartan medoxomil potassium-amlodipine final mixed granules were tableted using a tablet press to give azilsartan medoxomil potassium-amlodipine tablets.

### Example 7

### 1) Formula composition

| Name | Feeding amount per tablet (mg) | Percentage (%) |
|---|---|---|
| Internal addition | | |
| Azilsartan medoxomil potassium | 42.68 | 21.34 |
| Mannitol | 101.99 | 51.00 |

| Adhesive | | |
|---|---|---|
| Fumaric acid | 1.75 | 0.88 |
| Sodium hydroxide | 0.65 | 0.33 |
| Hydroxypropylcellulose | 4 | 2.00 |

| External addition | | |
|---|---|---|
| Amlodipine besylate | 6.93 | 3.47 |
| Microcrystalline cellulose | 25 | 12.50 |
| Croscarmellose sodium | 15 | 7.50 |
| Magnesium stearate | 2 | 1.10 |

### 2) Preparation method

Adhesive preparation: A solution with a solid content of 4% was prepared from hydroxypropylcellulose, fumaric acid, sodium hydroxide, and purified water. The mixture was stirred homogeneously and set aside for later use. Premixing: Mannitol and azilsartan medoxomil potassium were placed in a hopper mixer and mixed for 20 min.

Granulation: The mixed mannitol and azilsartan medoxomil potassium were added to a fluidized bed. The inlet airflow rate and the inlet air temperature were set, and preheating was performed. The aqueous solution of hydroxypropylcellulose, fumaric acid, and sodium hydroxide was then added, and fluidized granulation was performed. After granulation, the material was discharged when the water content was less than 3.0%. The granulated material was sized using a 1.5-mm sieve. External final mix: The azilsartan medoxomil potassium dry granules were then added to a hopper mixer. Amlodipine besylate, microcrystalline cellulose, and croscarmellose sodium were added, and the materials were mixed for 10 min. The mixed material was sized again using a 1.5-mm sieve. Finally, magnesium stearate was added, and the materials were mixed for another 10 min to give azilsartan medoxomil potassium-amlodipine final mixed granules.

Tableting: Finally, the azilsartan medoxomil potassium-amlodipine final mixed granules were tableted using a tablet press to give azilsartan medoxomil potassium-amlodipine tablets.

### Example 8

### 2) Formula composition

| Name | Feeding amount per tablet (mg) | Percentage (%) |
|---|---|---|
| Internal addition | | |
| Azilsartan medoxomil potassium | 42.68 | 21.34 |
| Mannitol | 101.99 | 51.00 |
| Hydroxypropylcellulose | 4 | 2.00 |

| pH regulator | | |
|---|---|---|
| Fumaric acid | 1.75 | 0.88 |
| Sodium hydroxide | 0.65 | 0.33 |

| External addition | | |
|---|---|---|
| Amlodipine besylate | 6.93 | 3.47 |
| Microcrystalline cellulose | 25 | 12.50 |
| Croscarmellose sodium | 15 | 7.50 |
| Magnesium stearate | 2 | 1.10 |

### 2) Preparation method

pH regulator preparation: A solution with a solid content of 4% was prepared from fumaric acid, sodium hydroxide, and purified water. The mixture was stirred homogeneously and set aside for later use.

Premixing: Mannitol, azilsartan medoxomil potassium, and hydroxypropylcellulose were placed in a hopper mixer and mixed for 20 min. Granulation: The mixed mannitol and azilsartan medoxomil potassium were added to a fluidized bed. The inlet airflow rate and the inlet air temperature were set, and preheating was performed. The aqueous solution of hydroxypropylcellulose, fumaric acid, and sodium hydroxide was then added, and fluidized granulation was performed. After granulation, the material was discharged when the water content was less than 3.0%. The granulated material was sized using a 1.5-mm sieve. External final mix: The azilsartan medoxomil potassium dry granules were then added to a hopper mixer. Amlodipine besylate, microcrystalline cellulose, and croscarmellose sodium were added, and the materials were mixed for 10 min. The mixed material was sized again using a 1.5-mm sieve. Finally, magnesium stearate was added, and the materials were mixed for another 10 min to give azilsartan medoxomil potassium-amlodipine final mixed granules.

Tableting: Finally, the azilsartan medoxomil potassium-amlodipine final mixed granules were tableted using a tablet press to give azilsartan medoxomil potassium-amlodipine tablets.

### Example 9

### 1) Formula composition

| Name | Feeding amount per tablet (mg) | Percentage (%) |
|---|---|---|
| **Amlodipine layer** | | |
| Amlodipine besylate | 6.93 | 2.31 |
| Microcrystalline cellulose | 100 | 33.33 |
| Croscarmellose sodium | 11.07 | 3.69 |
| Magnesium stearate | 2 | 0.67 |

| **Azilsartan medoxomil potassium layer** | | |
|---|---|---|
| Azilsartan medoxomil potassium | 42.68 | 14.23 |
| Mannitol | 101.92 | 33.97 |
| Hydroxypropylcellulose | 4 | 1.33 |
| Fumaric acid | 1.75 | 0.58 |
| Sodium hydroxide | 0.65 | 0.22 |
| Microcrystalline cellulose | 15 | 5.00 |
| Croscarmellose sodium | 12 | 4.00 |
| Magnesium stearate | 2 | 0.67 |

### 2) Preparation method

Amlodipine layer final mixed material preparation:
Microcrystalline cellulose, croscarmellose sodium, and amlodipine besylate were placed into a hopper mixer and mixed for 20 min. Magnesium stearate was then added, and the materials were mixed for 5 min to give amlodipine final mixed granules.

Azilsartan medoxomil potassium layer final mixed material preparation:
Adhesive preparation: A solution with a solid content of 4% was prepared from fumaric acid, sodium hydroxide, and purified water. The mixture was stirred homogeneously and set aside for later use.

Mannitol, azilsartan medoxomil potassium, and hydroxypropylcellulose were added to a fluidized bed. The inlet airflow rate and the inlet air temperature were set, and preheating was performed. The aqueous solution of fumaric acid and sodium hydroxide was then added, and fluidized granulation was performed. After granulation, the material was discharged when the water content was less than 3.0%. The granulated material was sized using a 1.5-mm sieve. The azilsartan medoxomil potassium dry granules were then added to a hopper mixer. Microcrystalline cellulose and croscarmellose sodium were added, and the materials were mixed for 10 min. Magnesium stearate was then added, and the materials were mixed for another 10 min to give azilsartan medoxomil potassium final mixed granules.

The amlodipine final mixed material and the azilsartan medoxomil potassium final mixed granules were tableted using a double-layer tablet press to give azilsartan medoxomil potassium-amlodipine double-layer tablets.

### Example 10

### 1) Formula composition

### 1) Formula composition

| Name | Feeding amount per tablet (mg) | Percentage (%) |
|---|---|---|
| Internal addition | 42.68 | 21.34 |
| Mannitol | 101.99 | 51.00 |
| Hydroxypropylcellulose | 4 | 2.00 |

| pH regulator | | |
|---|---|---|
| Fumaric acid | 1.75 | 0.88 |
| Sodium hydroxide | 0.65 | 0.33 |

| External addition | | |
|---|---|---|
| Amlodipine besylate | 6.93 | 3.47 |
| Microcrystalline cellulose | 25 | 12.50 |
| Croscarmellose sodium | 15 | 7.50 |
| Magnesium stearate | 2 | 1.10 |

### 2) Preparation method

pH regulator preparation: A solution with a solid content of 4% was prepared from fumaric acid, sodium hydroxide, and purified water. The mixture was stirred homogeneously and set aside for later use.

Premixing: Mannitol, azilsartan medoxomil potassium, and hydroxypropylcellulose were placed in a hopper mixer and mixed for 20 min. Granulation: The mixed mannitol and azilsartan medoxomil potassium were added to a fluidized bed. The inlet airflow rate and the inlet air temperature were set, and preheating was performed. The aqueous solution of hydroxypropylcellulose, fumaric acid, and sodium hydroxide was then added, and fluidized granulation was performed. After granulation, the material was discharged when the water content was less than 3.0%. The granulated material was sized using a 1.5-mm sieve. External final mix: The azilsartan medoxomil potassium dry granules were then added to a hopper mixer. Amlodipine besylate, microcrystalline cellulose, and croscarmellose sodium were added, and the materials were mixed for 10 min. The mixed material was sized again using a 1.5-mm sieve. Finally, magnesium stearate was added, and the materials were mixed for another 10 min to give azilsartan medoxomil potassium-amlodipine final mixed granules.

Capsule filling: Finally, the azilsartan medoxomil potassium-amlodipine final mixed granules were filled into No. 1 capsules to give azilsartan medoxomil potassium-amlodipine capsules.

### Test Example 1: Dissolution Property Testing

The dissolution test results of azilsartan medoxomil potassium in the formulas in Examples 5-10 in a phosphate buffer medium at pH 7.2 (900 mL) by the paddle method at 75 rpm are summarized in the table below and FIG. 1:

| Sample | Example 5 | Example 6 | Example 7 | Example 8 | Example 9 | Example 10 |
|---|---|---|---|---|---|---|
| Medium | Phosphate buffer, pH 7.2 | | | | | |
| Time (min) | Cumulative dissolution rate (%) | | | | | |
| 5 | 45 | 47 | 61 | 57 | 63 | 29 |
| 10 | 53 | 58 | 66 | 66 | 72 | 57 |
| 15 | 59 | 64 | 71 | 72 | 77 | 64 |
| 20 | 63 | 69 | 75 | 76 | 81 | 67 |
| 30 | 69 | 75 | 78 | 82 | 84 | 73 |
| 45 | 76 | 82 | 83 | 88 | 89 | 78 |
| 60 | 81 | 87 | 87 | 91 | 91 | 82 |

As can be seen from the dissolution results above, azilsartan medoxomil potassium in Examples 7, 8, and 9 exhibited relatively good dissolution rates and dissolution performance.

### Test Example 2: Stability Testing

Based on the dissolution results above, the azilsartan medoxomil potassium-amlodipine tablets obtained in Example 8 were subjected to stability testing after the tablets were packaged in high-density polyethylene bottles for oral solid dosage forms. The related substances were determined using the predetermined HPLC method. The test conditions and results are shown as follows.

| Conditions for standing | Known total impurity (%) | Unknown total impurity (%) | Total impurity (%) |
|---|---|---|---|
| 0 days | 2.7 | Undetectable | 2.7 |
| Standing at a high temperature of 50 °C, 60% RH for 10 days | 3.3 | 0.1 | 3.4 |
| Standing at 25 °C, 60% RH for 10 days | 3.0 | 0.1 | 3.0 |
| Standing at 30 °C, 65% RH for 10 days | 2.9 | Undetectable | 2.9 |
| Standing at 40 °C, 75% RH for 1 month | 3.7 | Undetectable | 3.7 |
| Standing at 40 °C, 75% RH for 2 months | 3.6 | Undetectable | 3.6 |

As can be seen from the stability results above, the tablets in Example 8 demonstrated relatively good stability with slow impurity growth. Therefore, the azilsartan medoxomil potassium-amlodipine tablets obtained in the present disclosure exhibit relatively good dissolution characteristics and good stability.

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A pharmaceutical composition, comprising azilsartan medoxomil potassium and a calcium channel blocker,
wherein:
the azilsartan medoxomil potassium comprises one or more of azilsartan medoxomil potassium, a pharmaceutically acceptable salt thereof, and a solvate thereof; and
the calcium channel blocker comprises one or more of a calcium channel blocker, a pharmaceutically acceptable salt thereof, and a solvate thereof.

2. The pharmaceutical composition according to claim 1, wherein the calcium channel blocker is one or more of amlodipine, levamlodipine, and a pharmaceutically acceptable salt thereof;
and/or
a dose of azilsartan medoxomil potassium comprised in the pharmaceutical composition is 20 mg-80 mg;
and/or
a dose of the calcium channel blocker in the pharmaceutical composition is 2.5 mg-20 mg.

3. The pharmaceutical composition according to claim 2, wherein a content of azilsartan medoxomil potassium in the pharmaceutical composition is 20 mg, 40 mg, or 80 mg;
and/or
a content of amlodipine in the pharmaceutical composition is 2.5 mg-20 mg;
and/or
a content of levamlodipine in the pharmaceutical composition is 2.5 mg-10 mg.

4. The pharmaceutical composition according to claim 1, further comprising one or more of a pH regulator, a diluent, an adhesive, a disintegrant, a lubricant, a glidant, a tableting aid, a colorant, and a coating premix.

5. The pharmaceutical composition according to claim 4, wherein the pH regulator is selected from one or more of fumaric acid, sodium fumarate, citric acid, sodium citrate, tartaric acid, sodium hydroxide, potassium dihydrogen phosphate, sodium dihydrogen phosphate, dicalcium phosphate, and sodium carbonate;
and/or
the diluent is selected from one or more of mannitol, anhydrous dicalcium phosphate, sucrose, glucose, maltose, lactose (e.g., lactose monohydrate), microcrystalline cellulose, and sorbitol;
and/or
the adhesive is selected from one or more of hydroxypropylcellulose, hydroxypropyl methylcellulose, hydroxyethylcellulose, copovidone, povidone, and pregelatinized starch;
and/or
the disintegrant is selected from one or more of sodium carboxymethyl starch, croscarmellose sodium, low-substituted hydroxypropylcellulose, crospovidone, and corn starch;
and/or
the lubricant is selected from one or more of a metal stearate (e.g., magnesium stearate), stearic acid, talc, a stearic acid ester, stearyl fumarate, and micronized silica gel;
and/or
the glidant is selected from one or more of talc, micronized silica gel, colloidal silicon dioxide, and pregelatinized starch;
and/or
the tableting aid is selected from microcrystalline cellulose and/or lactose monohydrate;
and/or
the colorant is selected from iron oxide red and/or iron oxide yellow;
and/or
the coating premix is a film coating premix provided by Colorcon.

6. The pharmaceutical composition according to claim 1, comprising azilsartan medoxomil potassium and amlodipine, or azilsartan medoxomil potassium and levamlodipine.

7. The pharmaceutical composition according to claim 6, wherein the pharmaceutical composition is an azilsartan medoxomil potassium-amlodipine tablet;
and/or
the pharmaceutical composition is an azilsartan medoxomil potassium-levamlodipine tablet.

8. A preparation method for the pharmaceutical composition according to any one of claims 1-7, comprising subjecting components of the pharmaceutical composition to one of a direct powder compression method, a wet granulation method, a fluidized granulation method, a dry granulation method, an extrusion-spheronization method, and a pellet coating method to prepare the pharmaceutical composition.

9. Use of the pharmaceutical composition according to any one of claims 1-7 for manufacturing a medicament for use in the treatment and/or prevention of a cardiovascular disease,
wherein preferably, the cardiovascular disease is selected from, for example, at least one of hypertension, heart failure, coronary heart disease, angina, arrhythmia, myocardial infarction, congenital heart disease, heart valve disease, and the like.

10. The use according to claim 9, wherein the medicament is a pharmaceutical formulation, preferably an oral pharmaceutical formulation;
for example, a dosage form of the pharmaceutical formulation is selected from a compound tablet, a double-layer tablet, a capsule, a pellet, a micro-tablet, and the like.
